# EUROPEAN PATENT SPECIFICATION

(11) **EP 4 501 237 B1**
(45) Date of publication and mention of the grant of the patent: **23.09.2026**
(21) Application number: 23779011.8
(22) Date of filing: 17.02.2023
(51) Int. Cl.: A61B 5/346

(54) **ELECTROCARDIOGRAM SIGNAL ANALYSIS SYSTEM**
ELEKTROKARDIOGRAMMSIGNALANALYSESYSTEM
SYSTÈME D'ANALYSE DE SIGNAUX D'ÉLECTROCARDIOGRAMME

(30) Priority: 29.03.2022 JP 2022053012
(43) Date of publication of application: 05.02.2025
(73) Proprietor: Toray Industries, Inc., Tokyo 103-8666 (JP)
(72) Inventor: OKAMOTO, Riku, Otsu-shi, Shiga 520-8558 (JP)
(74) Representative: Kador & Partner Part mbB
(86) International application number: PCT/JP2023/005850
(87) International publication number: WO 2023/188981

(56) References cited:
- CN-A- 113 209 477
- JP-A- 2000 014 653
- JP-A- 2000 014 653
- JP-A- 2018 175 152
- JP-A- 2019 005 305
- JP-A- 2021 047 578
- KR-A- 20130 048 596
- US-A- 5 178 154
- US-A1- 2021 236 827
- AIGUANG LI ET AL: "A novel abnormal ECG beats detection method", COMPUTER AND AUTOMATION ENGINEERING (ICCAE), 2010 THE 2ND INTERNATIONAL CONFERENCE ON, IEEE, PISCATAWAY, NJ, USA, 26 February 2010 (2010-02-26), pages 47 - 51, XP031661108, ISBN: 978-1-4244-5585-0
- ORPHANIDOU CHRISTINA ET AL: "Signal-Quality Indices for the Electrocardiogram and Photoplethysmogram: Derivation and Applications to Wireless Monitoring", IEEE JOURNAL OF BIOMEDICAL AND HEALTH INFORMATICS, IEEE, PISCATAWAY, NJ, USA, vol. 19, no. 3, 1 May 2015 (2015-05-01), pages 832 - 838, XP011580798, ISSN: 2168-2194, [retrieved on 20150508], DOI: 10.1109/JBHI.2014.2338351

## Description

### Field

The present invention relates to an electrocardiographic signal analysis system that estimates a measurement state of electrocardiographic signals based on, for example, electrocardiographic signals (hereinafter, electrocardiographic information).

### Background

The heart functions as a pump that repeats contraction and expansion to send blood throughout the body. This activity is regulated by weak electrical stimulation in myocardial cells, and occurrence of abnormality in this electrical stimulation also causes abnormality in the heart activity. This abnormality in the activity of the heart is called arrhythmia. There are various types of arrhythmias, and examples thereof include highly fatal ventricular fibrillation that causes cardiac arrest, ventricular tachycardia, and atrial fibrillation that causes cerebral infarction. Therefore, it is particularly essential to determine the arrhythmia, and the arrhythmia is determined using electrocardiographic information acquired from a determination target by an electrocardiographic signal measuring instrument.

The electrocardiographic information is obtained by measuring the potential of the heart by electrodes attached to the body surface of a subject. At this point, noise is superimposed on acquired electrocardiographic information due to floating of the electrodes caused by the body motion or influence of external disturbance. In particular, in a wearable electrocardiograph adopting a dry electrode such as "hitoe" (registered trademark), floating or the like of electrodes may occur rather easily, and thus noise may be superimposed rather frequently. Electrocardiographic information on which such noise is superimposed has lost characteristics as electrocardiographic information and becomes an obstacle in performing arrhythmia determination and the like, and thus it is preferable to exclude the electrocardiographic information from determination targets in advance as abnormal data, namely, noise data.

For example, Patent Literature 1 describes a method of generating data for arrhythmia determination (corresponding to the electrocardiographic information) excluding abnormal data regarding measured pulse wave information, namely, data that cannot be used for blood pressure estimation. Specifically, in Patent Literature **1,** each pulse waveform is determined to be abnormal data if it is not similar to the set reference data, using the similarity index to the set reference data.

In addition, Patent Literature 2 describes a method for estimating the quality of measured electrocardiographic information. Specifically, in Patent Literature 2, the degree of similarity to an adjacent waveform is used as an index, whereby the quality of the measured electrocardiographic information is estimated for each electrocardiographic waveform.
Patent Literature 3 discloses a steering wheel with an electrocardiographic signal detection device 1, a steering angle sensor 2, and a piezoelectric sensor 3. When the steering wheel is turned, the electrocardiographic signal detected is subjected to low-pass filter process. If a predetermined amount or more of pressure is detected by the piezoelectric sensor 3, that is, if the steering wheel is strongly gripped, a correlation coefficient between a template waveform and the electrocardiographic signal detected is calculated, and the section of the electrocardiographic signal where the value of the correlation coefficient is at or below a predetermined value is eliminated. The electrocardiographic signal detected is stored in a buffer 7, and if the action of the hand is detected by the piezoelectric sensor 3, the electrocardiographic signal stored in the buffer 7 is also subjected to correlation coefficient filter process. A myoelectric signal that has mixed into the electrocardiographic signal because of the movement of the wrist or the like before the piezoelectric sensor 3 detects the motion of the hand is also eliminated.
Patent Literature 4 discloses systems, devices, and methods for monitoring for atrial capture. Such a method, for use within an implantable system including an atrial leadless pacemaker (aLP) and a ventricular leadless pacemaker (vLP), includes storing within a memory of the vLP a paced atrial activation morphology template corresponding to far-field atrial signal components expected to be present in a vEGM sensed by the vLP when an atrial pacing pulse delivered by the aLP captures atrial tissue. The vLP senses a vEGM and compares a morphology of a portion of the sensed vEGM to the paced atrial activation morphology template to determine whether a match therebetween is detected. Additionally, the vLP determines whether atrial capture occurred or failed to occur (responsive to an atrial pacing pulse), based on whether the vLP detects a match between the morphology of a portion of the sensed vEGM and the paced atrial activation morphology template.
Patent Literature 5 discloses that parameters of cardiac activity can be derived from detecting thoracic impedance changes in a patient. An excitation signal is applied between a first pair electrodes located on the patient's forehead and torso. The voltage across another pair of electrodes positioned between the first pair is sensed to provide a first signal representing the impedance. The derivative of the first signal produces a second signal. Samples of the second signal taken during a number of cardiac cycles are ensemble averaged to minimized respiratory artifacts. Different criteria, such as the relationship between the second signal and an electrocardiographic signal, are employed to accept segments of the second signal which occur during different cardiac cycles. Samples of the accepted second signal segments for a number of cardiac cycles are ensemble averaged to minimize respiratory artifacts. The ensemble averaged data is employed to calculate cardiac stroke volume and cardiac output.
Non Patent Literature 1 relates to automatic detection of life threatening abnormal beats in electrocardiogram (ECG) signal. It discloses a two stage abnormal beats detection algorithm. Normal and abnormal beat types are represented by templates which are selected from training data using clustering. Multiple templates for each beat type well represent the distribution of the data and allow nonlinearity of the discrimination. Critical features are extracted for both templates and incoming beat signals in discrete wavelet transform domain. The template matching is carried out using time invariant dynamic time warping (DTW). For those the DTW distance cannot provide sharp discrimination, an additional stage of verification is invoked to further check among those types having small distance with the incoming beat, based on ECG wave's time interval features.
Non Patent Literature 2 discloses a signal quality index (SQI), which is intended to assess whether reliable heart rates (HRs) can be obtained from electrocardiogram (ECG) and photoplethysmogram (PPG) signals collected using wearable sensors. The algorithms were validated on manually labeled data. Additionally, it discloses two applications of the SQI. First, it is disclosed that by using the SQI as a trigger for a power-saving strategy, it is possible to reduce the recording time by up to 94% for the ECG and 93% for the PPG with only minimal loss of valid vital-sign data. Second, it is disclosed how an SQI can be used to reduce the error in the estimation of respiratory rate (RR) from the PPG.

### Citation List

### Patent Literature

Patent Literature 1: JP 2018-130319 A
Patent Literature 2: JP 2019-98183 A
Patent Literature 3: JP 2000 014653 A
Patent Literature 4: US 2021/236827 A1
Patent Literature 5: US 5 178 154 A

### Non Patent Literature

Non Patent Literature 1: AIGUANG LI ET AL: "A novel abnormal ECG beats detection method", COMPUTER AND AUTOMATION ENGINEERING (ICCAE), 2010 THE 2ND INTERNATIONAL CONFERENCE ON, IEEE, PISCATAWAY, NJ, USA, 26 February 2010 (2010-02-26), pages 47-51.
Non Patent Literature 2: ORPHANIDOU CHRISTINA ET AL: "Signal-Quality Indices for the Electrocardiogram and Photoplethysmogram: Derivation and Applications to Wireless Monitoring", IEEE JOURNAL OF BIOMEDICAL AND HEALTH INFORMATICS, IEEE, PISCATAWAY, NJ, USA, vol. 19, no. 3, 1 May 2015 (2015-05-01), pages 832-838,

### Summary

### Technical Problem

Meanwhile, the arrhythmia has various shapes of represented waveform depending on a type, and the shape and the timing of occurrence of the waveform are often different from those of normal sinus rhythm which is a typical electrocardiographic waveform. In each electrocardiographic waveform, an obtained shape may be deformed depending on a state of the electrocardiographic signal measuring instrument or the subject, and examples of the deformation include fluctuation in the wave height. However, since this deformed waveform has not lost the characteristics of the electrocardiographic waveform unlike the abnormal data described above, it can be used for arrhythmia determination.

However, the methods described in Patent Literatures 1 and 2 do not take into consideration various waveforms of arrhythmia and deformation of the waveform. Therefore, only by applying Patent Literature 1 or 2, a waveform of the arrhythmia or a deformed waveform in each electrocardiographic waveform may be erroneously determined as abnormal data, namely, noise data.

An object of the present invention is to provide the electrocardiographic signal analysis system capable of solving the above-described disadvantages of the conventional art and performing noise determination in consideration of various waveforms representing arrhythmia and deformed waveform.

### Solution to Problem

An electrocardiographic signal analysis system of the present invention that achieves the above object has the following configuration.
(1) An electrocardiographic signal analysis system that acquires electrocardiographic information and performs processing, the electrocardiographic signal analysis system including: an input unit configured to receive input of the electrocardiographic information; a filter processing unit configured to apply a filter to the electrocardiographic information and output filtered electrocardiographic information, the filter allowing a characteristic frequency to pass; a unit waveform data extracting unit configured to perform segmentation and extract unit waveform data based on a peak of an R wave or an S wave from waveform data included in the filtered electrocardiographic information; a data processing unit configured to generate processed data in which a wave height of the unit waveform data is adjusted; a reference data selecting unit configured to select reference data to be a reference of a measurement state from the processed data; a similarity calculation unit configured to calculate similarity between the reference data and the processed data of a determination target; and a determination unit configured to determine whether or not the electrocardiographic information corresponding to the processed data of the determination target includes noise based on the similarity. Embodiment (1) or "first embodiment" is not part of the invention. It is retained for illustrative purpose as all the features thereof are encompassed in the present invention.

The following embodiments (2) to (9) are of the invention. (2) The electrocardiographic signal analysis system that acquires electrocardiographic information and performs processing, the electrocardiographic signal analysis system including: an input unit configured to receive input of the electrocardiographic information; a filter processing unit configured to apply a filter to the electrocardiographic information and output filtered electrocardiographic information, the filter allowing a characteristic frequency to pass; a unit waveform data extracting unit configured to perform segmentation and extract unit waveform data based on a peak of an R wave or an S wave from waveform data included in the filtered electrocardiographic information; a data processing unit configured to generate processed data in which a wave height of the unit waveform data is adjusted; a reference data selecting unit configured to select reference data to be a reference of a measurement state from the processed data; a similarity calculation unit configured to calculate similarity between the reference data and the processed data of a determination target; and a determination unit configured to determine whether or not the electrocardiographic information corresponding to the processed data of the determination target includes noise based on the similarity, wherein concerning the unit waveform data, the data processing unit is configured to determine whether or not a potential of a minimum point immediately before or immediately after the peak of the R wave exceeds a reference or whether or not a potential of a maximum point immediately before or immediately after the peak of the S wave is below a reference to detect a peak loss, generate interpolation data in which the peak loss portion is interpolated, and adjust a wave height of the interpolation data.
(3) An electrocardiographic signal analysis system that acquires electrocardiographic information and performs processing, the electrocardiographic signal analysis system including: an input unit configured to receive input of the electrocardiographic information; a filter processing unit configured to apply a filter to the electrocardiographic information and output filtered electrocardiographic information, the filter allowing a characteristic frequency to pass; a unit waveform data extracting unit configured to perform segmentation and extract unit waveform data based on a peak of an R wave or an S wave from waveform data included in the filtered electrocardiographic information; a data processing unit configured to generate processed data in which a wave height of the unit waveform data is adjusted; a reference data selecting unit configured to select reference data to be a reference of a measurement state from the processed data; a similarity calculation unit configured to calculate similarity between the reference data and the processed data of a determination target; and a determination unit configured to determine whether or not the electrocardiographic information corresponding to the processed data of the determination target includes noise based on the similarity), wherein concerning the unit waveform data, the data processing unit is configured to detect determine whether or not a potential of a maximum point immediately before or immediately after the peak of the R wave exceeds a reference or whether or not a potential of a minimum point immediately before or immediately after the peak of the S wave is below a reference to detect a peak loss, generate interpolation data in which the peak loss portion is interpolated, and adjust a wave height of the interpolation data.
(4) The electrocardiographic signal analysis system that acquires electrocardiographic information and performs processing, the electrocardiographic signal analysis system including: an input unit configured to receive input of the electrocardiographic information; a filter processing unit configured to apply a filter to the electrocardiographic information and output filtered electrocardiographic information, the filter allowing a characteristic frequency to pass; a unit waveform data extracting unit configured to perform segmentation and extract unit waveform data based on a peak of an R wave or an S wave from waveform data included in the filtered electrocardiographic information; a data processing unit configured to generate processed data in which a wave height of the unit waveform data is adjusted; a reference data selecting unit configured to select reference data to be a reference of a measurement state from the processed data; a similarity calculation unit configured to calculate similarity between the reference data and the processed data of a determination target; and a determination unit configured to determine whether or not the electrocardiographic information corresponding to the processed data of the determination target includes noise based on the similarity, wherein concerning the unit waveform data, the data processing unit is configured to determine whether or not a potential of a point of inflection immediately before or immediately after the peak of the R wave exceeds a reference or whether or not a potential of a point of inflection immediately before or immediately after the peak of the S wave is below a reference to detect a peak loss, generate interpolation data in which the peak loss portion is interpolated, and adjust a wave height of the interpolation data.
(5) An electrocardiographic signal analysis system that acquires electrocardiographic information and performs processing, the electrocardiographic signal analysis system including: an input unit configured to receive input of the electrocardiographic information; a filter processing unit configured to apply a filter to the electrocardiographic information and output filtered electrocardiographic information, the filter allowing a characteristic frequency to pass; a unit waveform data extracting unit configured to perform segmentation and extract unit waveform data based on a peak of an R wave or an S wave from waveform data included in the filtered electrocardiographic information; a data processing unit configured to generate processed data in which a wave height of the unit waveform data is adjusted; a reference data selecting unit configured to select reference data to be a reference of a measurement state from the processed data; a similarity calculation unit configured to calculate similarity between the reference data and the processed data of a determination target; and a determination unit configured to determine whether or not the electrocardiographic information corresponding to the processed data of the determination target includes noise based on the similarity, wherein the data processing unit is configured to invert a wave height of the unit waveform data or the processed data, and adjust a wave height of the inverted data.
(6) An electrocardiographic signal analysis system that acquires electrocardiographic information and performs processing, the electrocardiographic signal analysis system including: an input unit configured to receive input of the electrocardiographic information; a filter processing unit configured to apply a filter to the electrocardiographic information and output filtered electrocardiographic information, the filter allowing a characteristic frequency to pass; a unit waveform data extracting unit configured to perform segmentation and extract unit waveform data based on a peak of an R wave or an S wave from waveform data included in the filtered electrocardiographic information; a data processing unit configured to generate processed data in which a wave height of the unit waveform data is adjusted; a reference data selecting unit configured to select reference data to be a reference of a measurement state from the processed data; a similarity calculation unit configured to calculate similarity between the reference data and the processed data of a determination target; and a determination unit configured to determine whether or not the electrocardiographic information corresponding to the processed data of the determination target includes noise based on the similarity, wherein the data processing unit is configured to invert time of the unit waveform data or the processed data, and adjust a wave height of the inverted data.
(7) The electrocardiographic signal analysis system according to the above aspects, wherein the similarity calculation unit is configured to calculate similarity between the reference data having been dynamically time-warped and the processed data of the determination target.
(8) The electrocardiographic signal analysis system according to any one of the above aspects, wherein the reference data selecting unit is configured to select the reference data from determination data generated based on electrocardiographic information acquired from a wearer wearing a measuring instrument that acquires the electrocardiographic information.
(9) The electrocardiographic signal analysis system according to any one of the above aspects, wherein the reference data selecting unit is configured to select the reference data from determination data generated based on electrocardiographic information acquired from a subject other than a wearer wearing a measuring instrument that acquires the electrocardiographic information.

### Advantageous Effects of Invention

According to the present invention, it is possible to perform noise determination in consideration of various waveforms representing arrhythmia or deformed waveform.

### Brief Description of Drawings

FIG. 1 is a diagram illustrating a configuration example of an electrocardiographic signal analysis system according to a first embodiment.
FIG. 2 is a diagram illustrating a detailed example of electrocardiographic information used for analysis.
FIG. 3 is a flowchart illustrating noise determination processing according to the first embodiment.
FIG. 4A is a diagram for describing a processing example of a filter processing unit according to the first embodiment (part 1).
FIG. 4B is a diagram for describing a processing example of the filter processing unit according to the first embodiment (part 2).
FIG. 4C is a diagram for describing a processing example of the filter processing unit according to the first embodiment (part 3).
FIG. 5 is a diagram illustrating a processing example of a unit waveform data extracting unit according to the first embodiment.
FIG. 6 is a diagram illustrating a processing example of a data processing unit according to the first embodiment.
FIG. 7 is a diagram for describing reference data selection processing according to the first embodiment.
FIG. 8 is a diagram illustrating a processing example of a similarity calculation unit according to the first embodiment.
FIG. 9 is a diagram for describing an example in which a waveform including noise is processed.
FIG. 10 is a diagram for describing processing of a data processing unit according to a second embodiment.
FIG. 11A is a diagram for describing a processing example of the data processing unit according to the second embodiment (part 1).
FIG. 11B is a diagram for describing a processing example of the data processing unit according to the second embodiment (part 2).
FIG. 11C is a diagram for describing a processing example of the data processing unit according to the second embodiment (part 3).
FIG. 12 is a diagram for describing processing of a data processing unit according to a third embodiment (part 1).
FIG. 13 is a diagram for describing processing of the data processing unit according to the third embodiment (part 2).

### Description of Embodiments

Hereinafter, embodiments of an electrocardiographic signal analysis system according to the present invention will be described in detail with reference to the drawings. Note that the present invention is not limited by the embodiments. Moreover, individual embodiments of the present invention are not independent and can be implemented in a combination as appropriate.

### (First Embodiment)

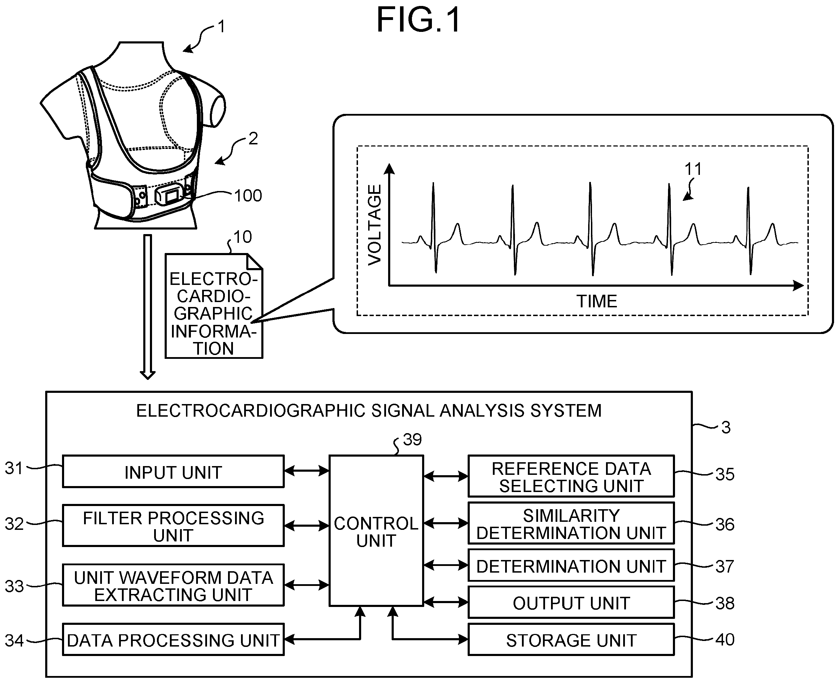

FIG. 1 is a diagram illustrating a configuration example of an electrocardiographic signal analysis system according to a first embodiment of the present invention. According to FIG. 1, electrocardiographic information 10 to be analyzed is acquired from a subject 1 via an electrocardiographic signal measuring instrument 2. The electrocardiographic information 10 herein indicates a minute amount of current generated with contraction and expansion of the heart. The subject 1 is not particularly limited such as a human or an animal.

Examples of the electrocardiographic signal measuring instrument 2 include a wearable electrocardiograph. Specifically, the electrocardiographic signal measuring instrument 2 includes a plurality of electrodes provided to the main body of an outfit worn by the subject 1 and an electrocardiograph 100 electrically connected to each of the electrodes.

The electrocardiograph 100 is an example of the electrocardiographic signal measuring instrument that measures an electrocardiographic signal of the subject. The electrocardiograph 100 has a function of continuously measuring an electrocardiographic signal (the electrocardiographic information 10) of the subject, a function of storing the obtained electrocardiographic information 10, and a function of transferring data by communication with the electrocardiographic signal analysis system 3. In addition to the above functions, the electrocardiograph 100 desirably has a control mechanism that starts measurement of the electrocardiographic information 10 simultaneously with attachment to the electrocardiographic signal measuring instrument 2. Examples of the measurement starting method include, but are not limited to, a method in which data measurement is started by connection to a dedicated connector provided in the electrocardiographic signal measuring instrument 2, and a method in which the electrocardiograph 100 starts data measurement with detection of a continuous electrocardiogram during intermittent measurement. According to this control mechanism, since the electrocardiographic information 10 is measured only when the electrocardiographic signal measuring instrument 2 is attached to the subject 1, it is possible to prevent wasteful power consumption and capacity consumption. Note that the electrocardiograph 100 may transfer data including the electrocardiographic information 10 to a server device, and the server device may transfer the electrocardiographic information 10 to the electrocardiographic signal analysis system 3. Hereinafter, description will be given on the premise that the subject 1 refers to a person wearing the electrocardiographic signal measuring instrument 2.

The electrocardiographic information 10 acquired from the subject 1 includes information of the subject 1, acquisition date and time, a location, and a measurement result. The measurement result is waveform data 11 obtained by plotting on the horizontal axis as time and the vertical axis as voltage (see FIG. 1).

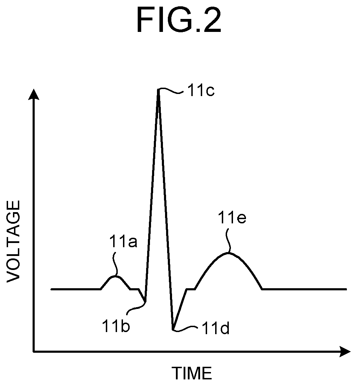

FIG. 2 is a diagram illustrating a detailed example of the electrocardiographic information 10 used for analysis. The waveform data 11 may include components referred to as a P wave 11a, a Q wave 11b, an R wave 11c, an S wave 11d, and a T wave 11e, and these shapes are used as determination criteria for arrhythmia. Note that the shape of the waveform data 11 included in the electrocardiographic information 10 may change depending on the subject or the position of the electrocardiograph 100 on the subject.

Returning back to FIG. 1, the electrocardiographic signal analysis system 3 according to the present invention includes an input unit 31, a filter processing unit 32, a unit waveform data extracting unit 33, a data processing unit 34, a reference data selecting unit 35, a similarity calculation unit 36, a determination unit 37, an output unit 38, a control unit 39, and a storage unit 40.

The input unit 31 receives input of the electrocardiographic information 10 output from the electrocardiographic signal measuring instrument 2. The input unit 31 includes a connector electrically connected to the electrocardiographic signal measuring instrument 2, a communication device including a communication means with the electrocardiographic signal measuring instrument 2, or an input port of a medium storing data and may further include a user interface such as a keyboard, a mouse, or a microphone.

The filter processing unit 32 performs filter processing on the waveform data 11 of the electrocardiographic information 10.

The unit waveform data extracting unit 33 extracts a unit waveform data 13 from waveform data having been subjected to filter processing.

The data processing unit 34 processes the unit waveform data 13 into a determination data 14.

The reference data selecting unit 35 selects data (reference data) to be used as a reference for noise determination from the determination data 14 generated by the data processing unit 34.

The similarity calculation unit 36 calculates the similarity between the reference data and the determination data 14 acquired from a determination target.

The determination unit 37 determines whether or not the electrocardiographic information 10 includes noise based on the similarity. The determination unit 37 determines the presence or absence of noise by comparing the similarity with a preset threshold. Set as the threshold is a boundary value for determining the presence or absence of noise concerning the similarity.

The output unit 38 includes an output device such as a display device or a printing device and outputs various types of information under the control by the control unit 39. The output unit 38 has, for example, a sound output function of a display including liquid crystal, organic electro luminescence (EL), or the like, a speaker, or the like.

The control unit 39 integrally controls the operation of the electrocardiographic signal analysis system 3. In addition, the control unit 39 causes the display to display the determination result of the determination unit 37, the electrocardiographic information 10, or information of normality and arrhythmia determined based on the electrocardiographic information 10 or outputs the information to the outside.

The storage unit 40 stores various programs for operating the electrocardiographic signal analysis system 3 or various types of data including data generated by the units. The various programs also include a determination program executed using a learning model. The storage unit 40 includes a read only memory (ROM) in which various programs and the like are installed in advance, a random access memory (RAM) that stores calculation parameters, data, and the like of each processing, a hard disk drive (HDD), a solid state drive (SSD), and the like.

The various programs can be recorded on a computer-readable recording medium such as an HDD, a flash memory, a CD-ROM, a DVD-ROM, or Blu-ray (registered trademark) and widely distributed. Furthermore, the input unit 31 can acquire the various programs via a communication network. The communication network mentioned here is configured using, for example, an existing public line network, a local area network (LAN), a wide area network (WAN), or the like and may be either wired or wireless.

The electrocardiographic signal analysis system 3 having the above functional configuration is a computer configured using one or a plurality of pieces of hardware such as a central processing unit (CPU), a graphics processing unit (GPU), an application specific integrated circuit (ASIC), and a field programmable gate array (FPGA).

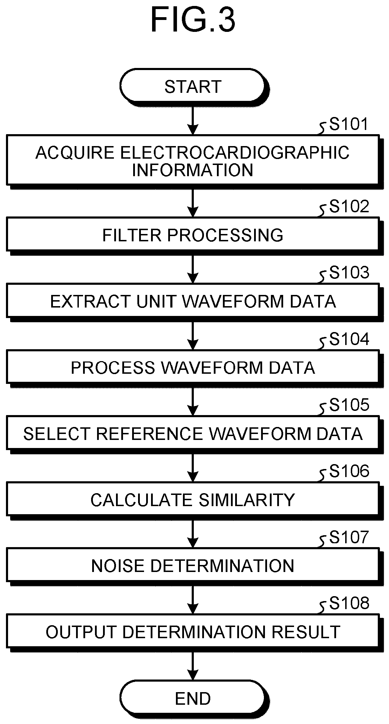

Next, noise determination processing based on the electrocardiographic information 10 obtained from the subject 1 will be described. FIG. 3 is a flowchart illustrating the noise determination processing according to the first embodiment. In the noise determination processing, first, the input unit 31 acquires the electrocardiographic information 10 obtained from the subject 1 via the electrocardiographic signal measuring instrument 2 (step S101).

The filter processing unit 32 performs filter processing on the waveform data 11 acquired in step S101 (step S102). For example, the filter processing unit 32 reduces the difference between the normal sinus rhythm and arrhythmia by allowing only the features of the electrocardiographic signal to pass from the waveform data 11 indicating the relationship between time and intensity (here, voltage), thereby making a difference from the noise obvious. Specifically, the filter processing unit 32 applies a frequency filter to the electrocardiographic information 10 input by the input unit 31 to generate filtered electrocardiographic information (waveform data) in which only characteristic frequencies of the normal sinus rhythm and the arrhythmia are allowed to pass. As a specific pass band of the frequency filter, 5 to 50 Hz or the like is set; however, it is not limited thereto. The frequency filter is designed using a general infinite impulse response (IIR) filter or a finite impulse response (FIR) filter.

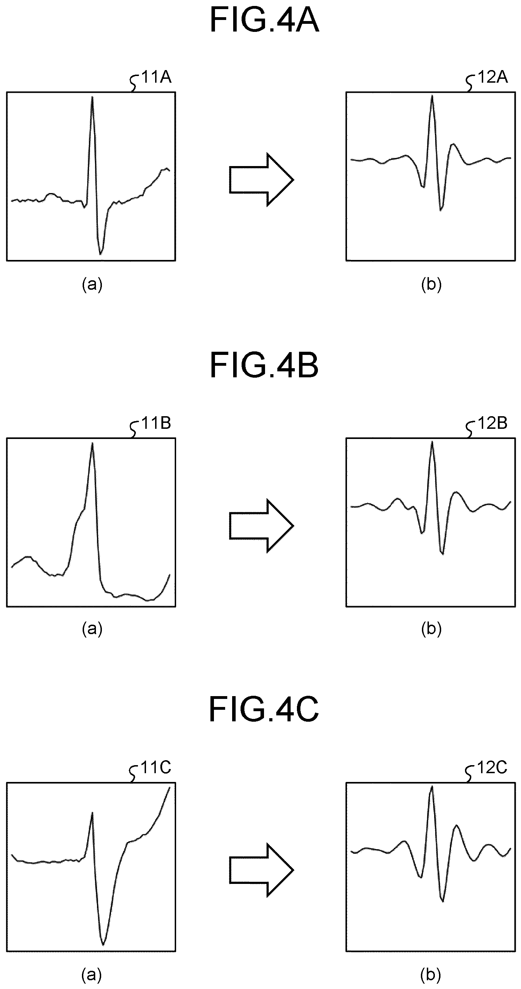

FIGS. 4A to 4C are diagrams for describing a processing example of the filter processing unit according to the first embodiment. Executing filter processing on waveform data 11A to 11C illustrated in (a) of FIGS. 4A to 4C results in generation of filtered waveform data 12A to 12C (see (b) of FIGS. 4A to 4C). The generated filtered waveform data 12 is registered in the storage unit 40.

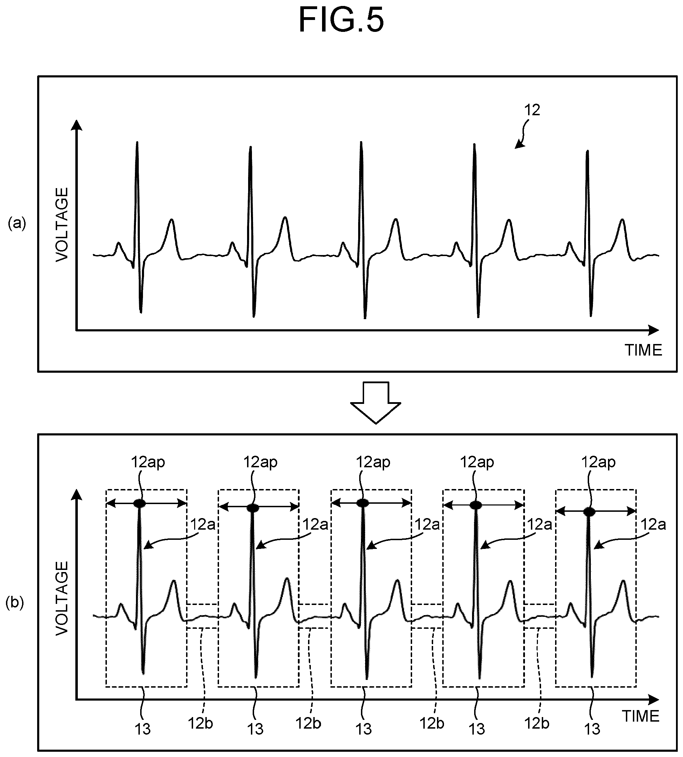

After the filter processing, the unit waveform data extracting unit 33 generates the unit waveform data 13 obtained by partial extraction from waveform data 12 (step S103). FIG. 5 is a diagram illustrating a processing example of the unit waveform data extracting unit according to the first embodiment. The unit waveform data extracting unit 33 generates the unit waveform data 13 in which each designated unit is extracted from the waveform data 12 illustrated in (a) of FIG. 5 (see (b) of FIG. 5) and stores the unit waveform data 13 in the storage unit 40. It is conceivable that extraction of the unit waveform data 13 is performed using, for example, a designated period based on time axis information as a unit or using a designated number of waveforms from a peak position of an R wave 12a as an extraction reference position; however, it is not limited thereto. In the example illustrated in FIG. 5, a peak position 12ap of the R wave 12a is detected, and a designated number of seconds before and after the peak position 12ap of the R wave 12a is set as one unit, whereby the unit waveform data 13 represents a single waveform based on the waveform data 12.

Incidentally, in the waveform data 12 (or the waveform data 11), intervals between the pieces of unit waveform data 13 is not constant due to heart rate fluctuation. However, with segmentation into each unit waveform, an effect of improving the accuracy of data processing by the data processing unit 34 and an effect of improving the accuracy of arrhythmia determination can be expected. In addition, since a section having no useful information for determination, such as out-of-unit waveform data 12b in (b) of FIG. 5, is excluded, the processing amount can be suppressed.

In addition, in the example illustrated in FIG. 5, in order to perform segmentation into unit waveforms, the designated number of seconds before and after (for example, 0.4 seconds) the peak position 12ap of the R wave 12a detected from the maximum value in a certain section is set as one unit waveform; however, it is not limited thereto. In a case where the waveform data 11 does not have the R wave 11c and the peak position 12ap of the R wave 12a cannot be detected, the unit waveform data extracting unit 33 obtains the maximum value of the waveform data 12 and, using it as a reference position, extracts the specified number of seconds before and after the reference position as one unit.

The data processing unit 34 processes the unit waveform data 13 into the determination data 14 (step S104). In the first embodiment, the data processing unit 34 generates the determination data 14 by adjusting the wave height as described above. The data processing unit 34 generates the determination data 14 in which the wave height is adjusted for the unit waveform data 13. The adjustment of the wave height is processing of correcting the wave height of pieces of unit waveform data 13 to be approximately equivalent to each other, whereby the influence of a difference in the wave height of each unit waveform becomes negligible when the determination unit 37 determines noise from the determination data 14. As a method of adjusting the wave height, a method using an R wave peak and an S wave peak, a method using an R wave peak and an offset amount, and the like are assumed, but it is not limited thereto. The data processing unit 34 stores the generated determination data 14 in the storage unit 40.

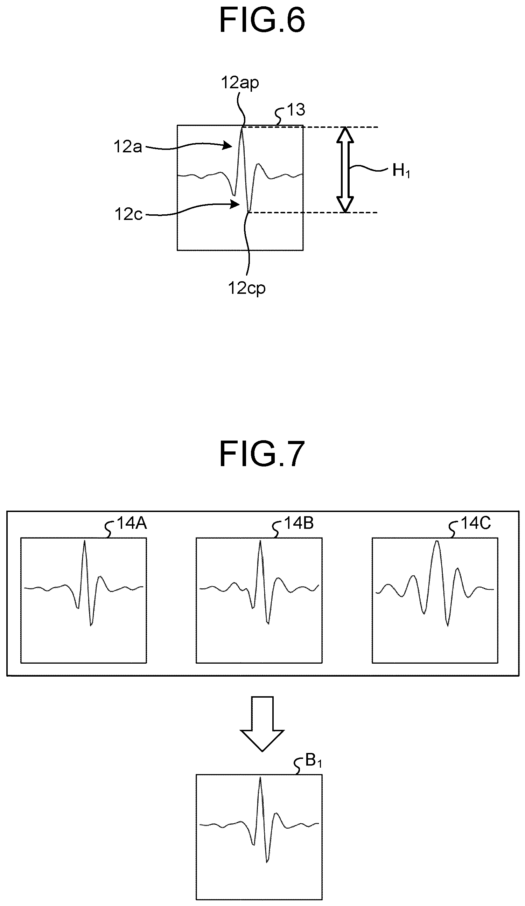

FIG. 6 is a diagram illustrating a processing example of the data processing unit according to the first embodiment. The data processing unit 34 adjusts a voltage width (wave height) H₁ from a peak position 12ap of an R wave 12a to a peak position 12cp of an S wave 12c in the unit waveform data 13.

After generation of the determination data 14, the reference data selecting unit 35 selects determination data to be reference data from the generated determination data 14 (step S105). The reference data has a role of enabling relative evaluation when the determination unit 37 determines whether or not the determination data 14 includes noise. Specifically, the reference data is presumed to be a normal sinus rhythm or types of arrhythmias, namely, an electrocardiographic waveform, and is used to determine whether or not noise is included in the determination data 14 through comparison with the determination data 14. For example, it is preferable to select determination data based on electrocardiographic information measured in a state where the electrocardiographic signal measuring instrument 2 is worn in accordance with an appropriate wearing procedure or determination data based on electrocardiographic information measured in a state where the electrocardiographic signal measuring instrument 2 is worn on the subject 1 with the assistance of an operator such as a doctor. This selection may be, for example, electrocardiographic information at the time when a preset period of time has elapsed from the start of measurement, such as electrocardiographic information immediately after the start of measurement by the electrocardiograph 100.

FIG. 7 is a diagram illustrating reference data selection processing according to the first embodiment. The reference data selecting unit 35 selects determination data to be reference data B₁ from among determination data 14A to 14C generated by the data processing unit 34, for example.

Note that, in the first embodiment, description will be given based on the premise that the reference data is selected from determination data acquired from the subject 1 to be determined, namely, determination data of the same subject; however, determination data acquired from a source other than the determination target may also be included for the selection. Originally, the electrocardiographic information 10 obtained by the electrocardiographic signal measuring instrument 2 varies depending on the subject, and there are individual differences; however, by creating data in which the individual difference is reduced by the filter processing or the data processing described above, it is possible to handle data acquired from a source other than the determination target as the reference data.

Meanwhile, as reference data for a subject showing a tendency such as ventricular hypertrophy is preferably selected from determination data 14 acquired from the determination target. Since electrocardiographic information acquired from such the subject tends to be different from electrocardiographic information of a healthy subject to an extent exceeding individual differences, preparing unique reference data makes it possible to guarantee the determination accuracy.

The method of selecting reference data is not limited to the above, and an operator may select determination data based on electrocardiographic information selected by observing the electrocardiographic information 10 or select determination data based on electrocardiographic information obtained by averaging waveforms acquired in a preset period.

Note that the reference data selection processing may be executed after a preset number of pieces of determination data are acquired after the acquisition of the electrocardiographic information 10 is started, or reference data may be selected from a plurality of pieces of determination data including determination data based on electrocardiographic information, of a subject different from the subject 1, which has been acquired in the past.

After the reference data is selected, the similarity calculation unit 36 calculates the similarity between the determination data 14 and the reference data (step S106). The similarity calculation unit 36 calculates dynamic similarity as the similarity. The similarity here refers to a distance between the reference data and the determination data 14, and the farther this distance is, the lower the similarity is. Moreover, as the similarity is lower, it is suspected that noise is included in the determination data 14. Incidentally, the dynamic similarity refers to similarity calculated by partially expanding and contracting the reference data and the determination data 14 in the time direction. As a method of calculating the dynamic similarity, a known method such as dynamic time warping (DTW) is applicable. By using the dynamic similarity, it is possible to ignore the influence of the arrhythmia or a deviation in the time direction occurring in a deformed waveform, and thus, it is possible to perform the noise determination more accurately.

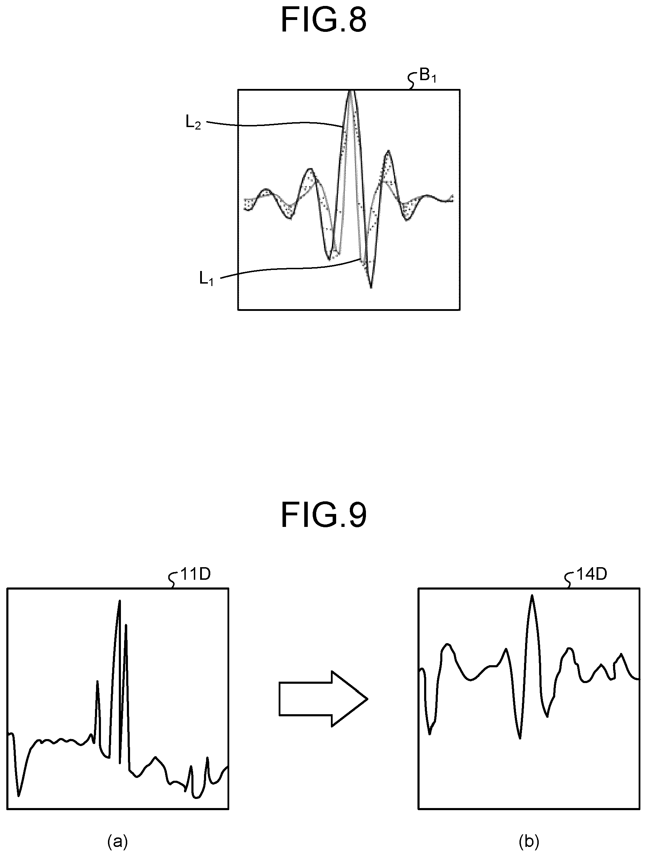

FIG. 8 is a diagram illustrating a processing example of the similarity calculation unit according to the first embodiment. For example, the similarity calculation unit 36 generates a waveform L₂ obtained by expanding and contracting the waveform L₁ of the reference data B₁ in the time axis direction. Note that points between the waveforms L₁ and L₂ indicate a locus for the waveform L₁ to be transformed to the waveform L₂. With regards to a warped waveform such as the waveform L2, the similarity calculation unit 36 calculates a distance using a warped waveform that is closest to the determination data 14.

FIG. 9 is a diagram for describing an example in which a waveform including noise is processed. Waveform data 11D illustrated in FIG. 9 includes noise, and a determination data 14D is generated by data processing. The determination data 14D has a long distance and low similarity. For example, in a case of determination data generated based on electrocardiographic information not including noise, the similarity is greater than or equal to 0.8, and in a case of determination data generated based on electrocardiographic information including noise, the similarity is less than 0.8. In this case, for example, the threshold for determination is set to 0.8.

Based on the similarity calculated in step S106, the determination unit 37 determines whether or not the electrocardiographic information 10 corresponding to the determination data 14 includes noise (step S107). The determination unit 37 compares the similarity with the threshold to determine whether or not noise is included and outputs the determination result.

The determination result as to whether or not noise is included that is output for each determination data is stored in the storage unit 40 by associating only the determination result as bool-type noise index data or as electrocardiographic information after replacement by replacing the noise portion with another value for the corresponding electrocardiographic information; however, it is not limited thereto.

After the noise determination, the control unit 39 outputs the determination result (step S108). If it is determined in step S107 that no noise is included, the control unit 39 outputs information indicating that no noise is included. On the other hand, if it is determined in step S107 that noise is included, the control unit 39 outputs information indicating that noise is included. The output unit 38 outputs sound or light to notify the determination result or causes a terminal held by the subject 1 or the operator to display the determination result. Furthermore, the control unit 39 may store the determination result in the storage unit 40.

Note that it is conceivable that the above-described determination processing is executed each time at timing at which new electrocardiographic information 10 is input or is executed each time at timing at which a certain amount of the electrocardiographic information 10 is accumulated; however, it is not limited thereto.

According to the first embodiment described above, the determination data 14 is generated by applying the filter processing of passing only the characteristic frequencies of the normal sinus rhythm and the arrhythmia to the waveform data 11 and adjusting the wave height, and the presence or absence of noise is determined by calculating the dynamic similarity based on the reference data. Therefore, it is possible to perform noise determination in consideration of various waveforms representing the arrhythmia or deformed waveform.

### (Second Embodiment)

Next, a second embodiment of the present invention will be described. Since the configuration of an electrocardiographic signal analysis system according to the second embodiment is the same as that of the electrocardiographic signal analysis system 3 according to the first embodiment, the description thereof will be omitted. Hereinafter, points different from those of the first embodiment will be described. The second embodiment is different from the first embodiment in the processing of the data processing unit 34. Note that the flow of the noise determination processing is similar to the flowchart illustrated in FIG. 3.

In the second embodiment, a data processing unit 34 detects a peak loss portion of an R wave or an S wave peak and interpolates data for the detected peak loss portion. The peak loss referred to herein means that a loss occurs at a peak of an R wave or an S wave of an obtained waveform due to a measurement failure of an electrocardiographic signal measuring instrument 2 or an influence of external disturbance. This peak loss portion contributes to reduce the similarity in a similarity calculation unit 36.

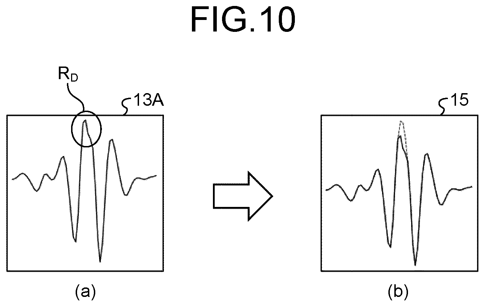

FIG. 10 is a diagram for describing processing of the data processing unit according to the second embodiment. The data processing unit 34 detects a peak loss portion of an R wave illustrated in a region R_{D} in a unit waveform data 13A (see (a) of FIG. 10) and generates an interpolation data 15 (see (b) of FIG. 10) obtained by interpolating data for the detected peak loss portion. In (b) of FIG. 10, the interpolated waveform is indicated by a broken line.

The data processing unit 34 first detects a peak loss portion in a determination data 14. As a method for detecting a peak loss, for example, an extreme value immediately before or after an R wave or S wave peak is used, and the extreme value is compared with a preset reference to detect a peak loss. Specifically, for example, a peak loss is detected by determining whether or not the potential of the minimum point immediately before or after an R wave peak exceeds the reference, or a peak loss is detected by determining whether or not the potential of the maximum point immediately before or after an R wave peak exceeds the reference. Similarly, for an S wave as well, a peak loss is detected by determining whether or not the potential of the maximum point immediately before or after an S wave peak is below the reference, or a peak loss is detected by determining whether or not the potential of the minimum point immediately before or after an S wave peak is below the reference. In this manner, a spike-like peak loss can be detected.

As a method of setting the reference for detecting a peak loss, for example, it is assumed that a baseline (zero point) of an electrocardiographic information 10 is used or that a value of 50% of the potential from the baseline to an R wave peak is used. However, an actual method is not specified, and it is desirable to appropriately adjust the value obtained using the above methods as a reference, for example.

Alternatively, as another method for detecting a peak loss, a point of inflection immediately before or after an R wave or S wave peak is used, and the potential at the point of inflection is compared with a preset reference to detect a peak loss. Specifically, for example, a peak loss is detected by determining whether or not the potential of the point of inflection immediately before or after an R wave peak exceeds the reference, or a peak loss is detected by determining whether or not the potential of the point of inflection immediately before or after an S wave peak is less than the reference. In this manner, a peak loss portion due to distortion can be detected.

Next, the data processing unit 34 interpolates data for the detected peak loss portion. As a method for interpolating the peak loss portion, a known method such as general spline interpolation can be adopted.

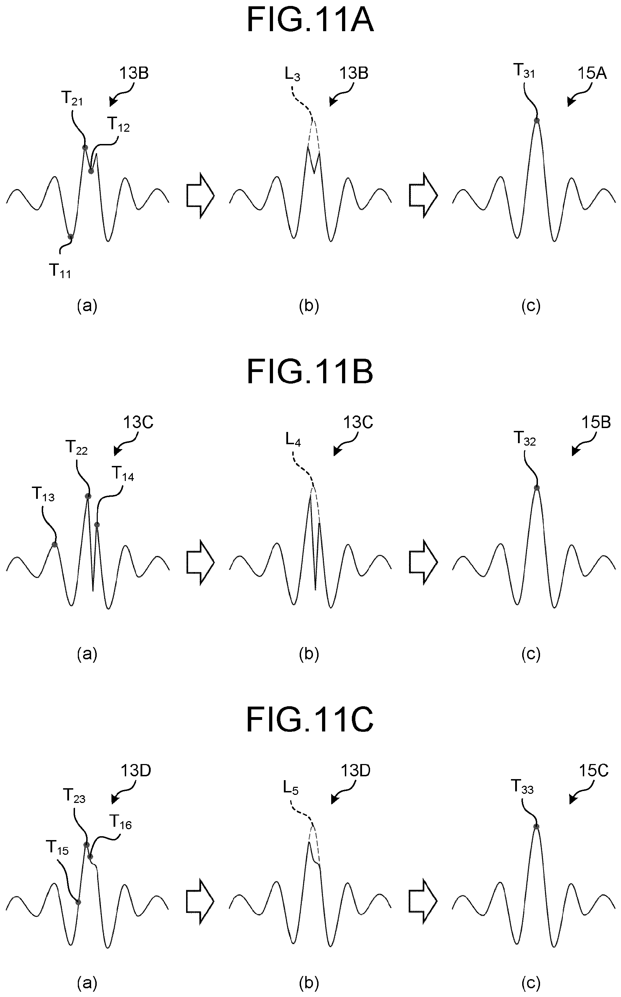

FIGS. 11A to 11C are diagrams for describing a processing example of the data processing unit according to the second embodiment. In FIGS. 11A to 11C, an interpolated waveform is indicated by a broken line. By the above-described peak loss detection and interpolation, an interpolation data 15 in which a loss portion is interpolated is obtained.

### [Case of Using Minimum Point Immediately Before or Immediately After Peak]

The data processing unit 34 detects, for example, a minimum point T₁₁ immediately before a peak of an R wave or a minimum point T₁₂ immediately after the peak of the R wave for a unit waveform data 13B and determines whether or not the potential of the minimum point exceeds a reference to detect a peak loss (see (a) of FIG. 11A). Then, the data processing unit 34 interpolates an interpolation waveform L₃ for the peak loss portion (see (b) of FIG. 11A) to generate an interpolation data 15A (see (c) of FIG. 11A). As a result, the peak position of the R wave shifts from a peak position T₂₁ to a peak position T₃₁.

### [Case of Using Maximum Point Immediately Before or Immediately After Peak]

The data processing unit 34 detects, for example, a maximum point T₁₃ immediately before a peak of an R wave or a maximum point T₁₄ immediately after the peak of the R wave for a unit waveform data 13C and determines whether or not the potential of the maximum point exceeds a reference to detect a peak loss (see (a) of FIG. 11B). Then, the data processing unit 34 interpolates an interpolation waveform L₄ for the peak loss portion (see (b) of FIG. 11B) and generates an interpolation data 15B (see (c) of FIG. 11B). As a result, the peak position of the R wave shifts from a peak position T₂₂ to a peak position T₃₂.

### [Case of Using Point of Inflection Immediately Before or Immediately After Peak]

The data processing unit 34 detects, for example, a point of inflection T₁₅ immediately before a peak of an R wave or a point of inflection T₁₆ immediately after the peak of the R wave for a unit waveform data 13D and determines whether or not the potential of the point of inflection exceeds a reference to detect a peak loss (see (a) of FIG. 11C). Then, the data processing unit 34 interpolates an interpolation waveform L₅ for the peak loss portion (see (b) of FIG. 11C) and generates an interpolation data 15C (see (c) of FIG. 11C). As a result, the peak position of the R wave shifts from a peak position T₂₃ to a peak position T₃₃.

After the interpolation processing, the data processing unit 34 adjusts the wave height of the interpolation data 15.

In the second embodiment described above, the filter processing of passing only the characteristic frequencies of the normal sinus rhythm and arrhythmia is performed on the waveform data 11 to interpolate for the peak loss portion, a determination data 14 in which the wave height of the interpolation data 15 is adjusted is generated, and dynamic similarity is calculated based on the reference data, whereby the presence or absence of noise is determined. According to the second embodiment, it is possible to perform noise determination in consideration of various waveforms representing arrhythmia or deformed waveform by applying filter processing or data processing.

### (Third Embodiment)

Next, a third embodiment of the present invention will be described. Since the configuration of an electrocardiographic signal analysis system according to the third embodiment is the same as that of the electrocardiographic signal analysis system 3 according to the first embodiment, the description thereof will be omitted. Hereinafter, points different from those of the first embodiment will be described. The third embodiment is different from the first embodiment in the processing of the data processing unit 34. Note that the flow of the noise determination processing is similar to the flowchart illustrated in FIG. 3.

Meanwhile, in a case where a similarity calculation unit 36 calculates the similarity between data in which the influence of an R wave is strong (hereinafter, referred to as R-type data) and data in which the influence of an S wave is strong (hereinafter, referred to as S-type data), the accuracy of the similarity calculation may be lower as compared to the similarity calculated between R-type data or between S-type data.

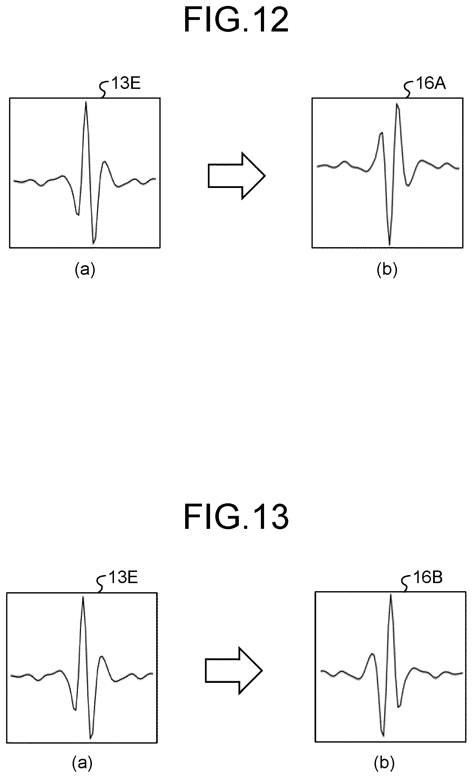

Therefore, in the third embodiment, a data processing unit 34 generates an inverted data 16 obtained by inverting at least one of the wave height or the time in a unit waveform data 13 or the determination data 14. FIGS. 12 and 13 are diagrams for describing processing of the data processing unit according to the third embodiment.

In the case of inverting the wave height, the data processing unit 34 generates, for example, an inverted data 16A obtained by inverting a wave height of a unit waveform data 13E (see FIG. 12). Incidentally, the inversion of the wave height is synonymous with processing of inverting the sign of the unit waveform data 13 or a determination data 14 in the amplitude direction (vertical axis). Therefore, the inverted data 16A in which the wave height is inverted can also be considered as the original antiphase data.

In the case of inverting the time, the data processing unit 34 generates, for example, an inverted data 16B obtained by inverting the time of the unit waveform data 13E (see FIG. 13). Incidentally, the inversion of the time is synonymous with processing of inverting the sign of the unit waveform data 13 or the determination data 14 in the time direction (horizontal axis). Therefore, the inverted data 16B in which the time is inverted can also be considered as reverse (reverse reproduction) data of the original data.

Furthermore, the data processing unit 34 may generate an inverted data 16C in which the wave height and the time are inverted. Note that a target to be inverted can be determined by setting, and data subjected to inversion processing and data not subjected to inversion processing can be included as the determination data 14.

In this case, a determination unit 37 may set different threshold values to a plurality of types of data. In addition, a plurality of determination results each output for one of various types of data is integrated into a logical sum, a logical product, or a combination thereof, and one determination result is finally output.

After the inversion processing, the data processing unit 34 adjusts the wave height of the inverted data 16.

In the third embodiment described above, the filter processing of passing only the characteristic frequencies of the normal sinus rhythm and arrhythmia is performed on waveform data 11 to invert at least one of the wave height or the time is, the determination data 14 in which the wave height is adjusted is generated, and dynamic similarity is calculated based on the reference data, whereby the presence or absence of noise is determined. According to the third embodiment, it is possible to perform noise determination in consideration of various waveforms representing arrhythmia or deformed waveform by applying filter processing or data processing.

Furthermore, according to the third embodiment, by generating the determination data 14 in which the wave height and the time are inverted, it is possible to calculate the similarity in the case of calculating the similarity between R-type data and S-type data with high accuracy.

### (Fourth Embodiment)

Next, a fourth embodiment of the present invention will be described. Since the configuration of an electrocardiographic signal analysis system according to the fourth embodiment is the same as that of the electrocardiographic signal analysis system 3 according to the first embodiment, the description thereof will be omitted. Hereinafter, points different from those of the first embodiment will be described.

In the fourth embodiment, in addition to the processing described above, a determination unit 37 calculates an electrocardiogram acquisition rate based on a determination result, and a output unit 38 outputs the electrocardiogram acquisition rate calculated as the determination result to a subject 1 or the operator. The electrocardiogram acquisition rate refers to a ratio of a non-noise section in a measurement time, namely, a section effective for arrhythmia determination. At this point, since the accuracy of arrhythmia determination or the signal acquisition efficiency are low for data having a low electrocardiogram acquisition rate, remeasurement is required in some cases.

According to the fourth embodiment, since a control unit 39 calculates and outputs the electrocardiogram acquisition rate, the subject 1 or the operator can immediately determine about remeasurement. Note that all or some of the functions of the electrocardiographic signal analysis system 3 may be included in an electrocardiograph 100.

### (Other Embodiments)

Although the embodiments for carrying out the present invention have been described above, the present invention should not be limited only by the above-described embodiments. For example, it has been described that the electrocardiographic signal analysis system 3 is provided separately from the electrocardiographic signal measuring instrument 2; however, the electrocardiographic signal analysis system 3 may be configured integrally with the electrocardiographic signal measuring instrument 2. In addition, at least some of the functions of the electrocardiographic signal analysis system 3 may be included in the electrocardiograph 100. For example, the electrocardiograph 100 may include the input unit 31, the filter processing unit 32, the unit waveform data extracting unit 33, the data processing unit 34, the reference data selecting unit 35, the similarity calculation unit 36, the determination unit 37, the output unit 38, and the control unit 39.

### Industrial Applicability

By using the electrocardiographic signal analysis system according to the present invention, it is possible to determine noise in consideration of various waveforms representing arrhythmia or deformed waveform.

### Reference Signs List

1 SUBJECT
2 ELECTROCARDIOGRAPHIC SIGNAL MEASURING INSTRUMENT
3 ELECTROCARDIOGRAPHIC SIGNAL ANALYSIS SYSTEM
10 ELECTROCARDIOGRAPHIC INFORMATION
11, 11A to 11D, 12, 12A to 12C WAVEFORM DATA
13, 13A to 13E UNIT WAVEFORM DATA
14, 14A to 14D DETERMINATION DATA
15, 15A to 15C INTERPOLATION DATA
16, 16A to 16C INVERTED DATA
31 INPUT UNIT
32 FILTER PROCESSING UNIT
33 UNIT WAVEFORM DATA EXTRACTING UNIT
34 DATA PROCESSING UNIT
35 REFERENCE DATA SELECTING UNIT
36 SIMILARITY CALCULATION UNIT
37 DETERMINATION UNIT
38 OUTPUT UNIT
39 CONTROL UNIT
40 STORAGE UNIT
100 ELECTROCARDIOGRAPH

## Claims

1. An electrocardiographic signal analysis system (3) that acquires electrocardiographic information and performs processing, the electrocardiographic signal analysis system comprising:
an input unit (31) configured to receive input of the electrocardiographic information;
a filter processing unit (32) configured to apply a filter to the electrocardiographic information and output filtered electrocardiographic information, the filter allowing a characteristic frequency to pass;
a unit waveform data extracting unit (33) configured tc perform segmentation and extract unit waveform data based on a peak of an R wave or an S wave from waveform data included in the filtered electrocardiographic information;
a data processing unit (34) configured to generate processed data in which a wave height of the unit waveform data is adjusted;
a reference data selecting unit (35) configured to select reference data to be a reference of a measurement state from the processed data;
a similarity calculation unit (36) configured to calculate similarity between the reference data and the processed data of a determination target; and
a determination unit (37) configured to determine whether or not the electrocardiographic information corresponding to the processed data of the determination target includes noise based on the similarity,
wherein
concerning the unit waveform data, the data processing unit is configured to
determine whether or not a potential of a minimum point immediately before or immediately after the peak of the R wave exceeds a reference or whether or not a potential of a maximum point immediately before or immediately after the peak of the S wave is below a reference to detect a peak loss,
generate interpolation data in which the peak loss portion is interpolated, and
adjust a wave height of the interpolation data.

2. An electrocardiographic signal analysis system (3) that acquires electrocardiographic information and performs processing, the electrocardiographic signal analysis system comprising:
an input unit (31) configured to receive input of the electrocardiographic information;
a filter processing unit (32) configured to apply a filter to the electrocardiographic information and output filtered electrocardiographic information, the filter allowing a characteristic frequency to pass;
a unit waveform data extracting unit (33) configured tc perform segmentation and extract unit waveform data based on a peak of an R wave or an S wave from waveform data included in the filtered electrocardiographic information;
a data processing unit (34) configured to generate processed data in which a wave height of the unit waveform data is adjusted;
a reference data selecting unit (35) configured to select reference data to be a reference of a measurement state from the processed data;
a similarity calculation unit (36) configured to calculate similarity between the reference data and the processed data of a determination target; and
a determination unit (37) configured to determine whether or not the electrocardiographic information corresponding to the processed data of the determination target includes noise based on the similarity, wherein
concerning the unit waveform data, the data processing unit is configured to
detect determine whether or not a potential of a maximum point immediately before or immediately after the peak of the R wave exceeds a reference or whether or not a potential of a minimum point immediately before or immediately after the peak of the S wave is below a reference to detect a peak loss,
generate interpolation data in which the peak loss portion is interpolated, and
adjust a wave height of the interpolation data.

3. An electrocardiographic signal analysis system (3) that acquires electrocardiographic information and performs processing, the electrocardiographic signal analysis system comprising:
an input unit (31) configured to receive input of the electrocardiographic information;
a filter processing unit (32) configured to apply a filter to the electrocardiographic information and output filtered electrocardiographic information, the filter allowing a characteristic frequency to pass;
a unit waveform data extracting unit (33) configured tc perform segmentation and extract unit waveform data based on a peak of an R wave or an S wave from waveform data included in the filtered electrocardiographic information;
a data processing unit (34) configured to generate processed data in which a wave height of the unit waveform data is adjusted;
a reference data selecting unit (35) configured to select reference data to be a reference of a measurement state from the processed data;
a similarity calculation unit (36) configured to calculate similarity between the reference data and the processed data of a determination target; and
a determination unit (37) configured to determine whether or not the electrocardiographic information corresponding to the processed data of the determination target includes noise based on the similarity, wherein
concerning the unit waveform data, the data processing unit is configured to
determine whether or not a potential of a point of inflection immediately before or immediately after the peak of the R wave exceeds a reference or whether or not a potential of a point of inflection immediately before or immediately after the peak of the S wave is below a reference to detect a peak loss,
generate interpolation data in which the peak loss portion is interpolated, and
adjust a wave height of the interpolation data.

4. An electrocardiographic signal analysis system (3) that acquires electrocardiographic information and performs processing, the electrocardiographic signal analysis system comprising:
an input unit (31) configured to receive input of the electrocardiographic information;
a filter processing unit (32) configured to apply a filter to the electrocardiographic information and output filtered electrocardiographic information, the filter allowing a characteristic frequency to pass;
a unit waveform data extracting unit (33) configured tc perform segmentation and extract unit waveform data based on a peak of an R wave or an S wave from waveform data included in the filtered electrocardiographic information;
a data processing unit (34) configured to generate processed data in which a wave height of the unit waveform data is adjusted;
a reference data selecting unit (35) configured to select reference data to be a reference of a measurement state from the processed data;
a similarity calculation unit (36) configured to calculate similarity between the reference data and the processed data of a determination target; and
a determination unit (37) configured to determine whether or not the electrocardiographic information corresponding to the processed data of the determination target includes noise based on the similarity, wherein
the data processing unit (34) is configured tc invert a wave height of the unit waveform data or the processed data, and
adjust a wave height of the inverted data.

5. An electrocardiographic signal analysis system (3) that acquires electrocardiographic information and performs processing, the electrocardiographic signal analysis system comprising:
an input unit (31) configured to receive input of the electrocardiographic information;
a filter processing unit (32) configured to apply a filter to the electrocardiographic information and output filtered electrocardiographic information, the filter allowing a characteristic frequency to pass;
a unit waveform data extracting unit (33) configured tc perform segmentation and extract unit waveform data based on a peak of an R wave or an S wave from waveform data included in the filtered electrocardiographic information;
a data processing unit (34) configured to generate processed data in which a wave height of the unit waveform data is adjusted;
a reference data selecting unit (35) configured to select reference data to be a reference of a measurement state from the processed data;
a similarity calculation unit (36) configured to calculate similarity between the reference data and the processed data of a determination target; and
a determination unit (37) configured to determine whether or not the electrocardiographic information corresponding to the processed data of the determination target includes noise based on the similarity, wherein
the data processing unit (34) is configured to invert time of the unit waveform data or the processed data, and
adjust a wave height of the inverted data.

6. The electrocardiographic signal analysis system according to any one of claims 1 to 5, wherein
the similarity calculation unit is configured to
calculate similarity between the reference data having been dynamically time-warped and the processed data of the determination target.

7. The electrocardiographic signal analysis system according to any one of claims 1 to 6, wherein
the reference data selecting unit is configured to select the reference data from determination data generated based on electrocardiographic information acquired from a wearer wearing a measuring instrument that acquires the electrocardiographic information.

8. The electrocardiographic signal analysis system according to any one of claims 1 to 6, wherein
the reference data selecting unit is configured to select the reference data from determination data generated based on electrocardiographic information acquired from a subject other than a wearer wearing a measuring instrument that acquires the electrocardiographic information.

## Patentansprüche

1. Elektrokardiogrammsignal-Analysesystem (3), das elektrokardiografische Informationen erhält und eine Verarbeitung durchführt, wobei das Elektrokardiogrammsignal-Analysesystem umfasst:
eine Eingabeeinheit (31), die konfiguriert ist zum Empfangen einer Eingabe der elektrokardiografischen Informationen,
eine Filterverarbeitungseinheit (32), die konfiguriert ist zum Anwenden eines Filters auf die elektrokardiografischen Informationen und zum Ausgeben von gefilterten elektrokardiografischen Informationen, wobei das Filter ein Hindurchgehen einer charakteristischen Frequenz erlaubt,
eine Einheitswellenformdaten-Extraktionseinheit (33), die konfiguriert ist zum Durchführen einer Segmentierung und zum Extrahieren von Einheitswellenformdaten basierend auf einer Spitze einer R-Welle oder einer S-Welle aus in den gefilterten elektrokardiografischen Informationen enthaltenen Wellenformdaten,
eine Datenverarbeitungseinheit (34), die konfiguriert ist zum Generieren von verarbeiteten Daten, in denen eine Wellenhöhe der Einheitswellenformdaten eingestellt ist,
eine Referenzdaten-Auswahleinheit (35), die konfiguriert ist zum Auswählen von Referenzdaten als einer Referenz eines Messzustands aus den verarbeiteten Daten,
eine Ähnlichkeitsberechnungseinheit (36), die konfiguriert ist zum Berechnen einer Ähnlichkeit zwischen den Referenzdaten und den verarbeiteten Daten eines Bestimmungsziels, und
eine Bestimmungseinheit (37), die konfiguriert ist zum Bestimmen, ob die elektrokardiografischen Informationen in Entsprechung zu den verarbeiteten Daten des Bestimmungsziels ein Rauschen enthalten oder nicht, basierend auf der Ähnlichkeit,
wobei, hinsichtlich der Einheitswellenformdaten, die Datenverarbeitungseinheit konfiguriert ist zum:
Bestimmen, ob ein Potential eines Minimumpunkts unmittelbar vor oder unmittelbar nach der Spitze der R-Welle eine Referenz überschreitet oder nicht oder ob ein Potential eines Maximumpunkts unmittelbar vor oder unmittelbar nach der Spitze der S-Welle unterhalb einer Referenz liegt oder nicht, für das Erfassen eines Spitzenverlusts,
Generieren von Interpolationsdaten, in denen der Spitzenverlustteil interpoliert ist, und
Einstellen einer Wellenhöhe der Interpolationsdaten.

2. Elektrokardiogrammsignal-Analysesystem (3), das elektrokardiografische Informationen erhält und eine Verarbeitung durchführt, wobei das Elektrokardiogrammsignal-Analysesystem umfasst:
eine Eingabeeinheit (31), die konfiguriert ist zum Empfangen einer Eingabe der elektrokardiografischen Informationen,
eine Filterverarbeitungseinheit (32), die konfiguriert ist zum Anwenden eines Filters auf die elektrokardiografischen Informationen und zum Ausgeben von gefilterten elektrokardiografischen Informationen, wobei das Filter ein Hindurchgehen einer charakteristischen Frequenz erlaubt,
eine Einheitswellenformdaten-Extraktionseinheit (33), die konfiguriert ist zum Durchführen einer Segmentierung und zum Extrahieren von Einheitswellenformdaten basierend auf einer Spitze einer R-Welle oder einer S-Welle aus in den gefilterten elektrokardiografischen Informationen enthaltenen Wellenformdaten,
eine Datenverarbeitungseinheit (34), die konfiguriert ist zum Generieren von verarbeiteten Daten, in denen eine Wellenhöhe der Einheitswellenformdaten eingestellt ist,
eine Referenzdaten-Auswahleinheit (35), die konfiguriert ist zum Auswählen von Referenzdaten als einer Referenz eines Messzustands aus den verarbeiteten Daten,
eine Ähnlichkeitsberechnungseinheit (36), die konfiguriert ist zum Berechnen einer Ähnlichkeit zwischen den Referenzdaten und den verarbeiteten Daten eines Bestimmungsziels, und
eine Bestimmungseinheit (37), die konfiguriert ist zum Bestimmen, ob die elektrokardiografischen Informationen in Entsprechung zu den verarbeiteten Daten des Bestimmungsziels ein Rauschen enthalten oder nicht, basierend auf der Ähnlichkeit,
wobei, hinsichtlich der Einheitswellenformdaten, die Datenverarbeitungseinheit konfiguriert ist zum:
Erfassen/Bestimmen, ob ein Potential eines Maximumpunkts unmittelbar vor oder unmittelbar nach der Spitze der R-Welle eine Referenz überschreitet oder nicht oder ob ein Potential eines Minimumpunkts unmittelbar vor oder unmittelbar nach der Spitze der S-Welle unterhalb einer Referenz liegt oder nicht, für das Erfassen eines Spitzenverlusts,
Generieren von Interpolationsdaten, in denen der Spitzenverlustteil interpoliert ist, und
Einstellen einer Wellenhöhe der Interpolationsdaten.

3. Elektrokardiogrammsignal-Analysesystem (3), das elektrokardiografische Informationen erhält und eine Verarbeitung durchführt, wobei das Elektrokardiogrammsignal-Analysesystem umfasst:
eine Eingabeeinheit (31), die konfiguriert ist zum Empfangen einer Eingabe der elektrokardiografischen Informationen,
eine Filterverarbeitungseinheit (32), die konfiguriert ist zum Anwenden eines Filters auf die elektrokardiografischen Informationen und zum Ausgeben von gefilterten elektrokardiografischen Informationen, wobei das Filter ein Hindurchgehen einer charakteristischen Frequenz erlaubt,
eine Einheitswellenformdaten-Extraktionseinheit (33), die konfiguriert ist zum Durchführen einer Segmentierung und zum Extrahieren von Einheitswellenformdaten basierend auf einer Spitze einer R-Welle oder einer S-Welle aus in den gefilterten elektrokardiografischen Informationen enthaltenen Wellenformdaten,
eine Datenverarbeitungseinheit (34), die konfiguriert ist zum Generieren von verarbeiteten Daten, in denen eine Wellenhöhe der Einheitswellenformdaten eingestellt ist,
eine Referenzdaten-Auswahleinheit (35), die konfiguriert ist zum Auswählen von Referenzdaten als einer Referenz eines Messzustands aus den verarbeiteten Daten,
eine Ähnlichkeitsberechnungseinheit (36), die konfiguriert ist zum Berechnen einer Ähnlichkeit zwischen den Referenzdaten und den verarbeiteten Daten eines Bestimmungsziels, und
eine Bestimmungseinheit (37), die konfiguriert ist zum Bestimmen, ob die elektrokardiografischen Informationen in Entsprechung zu den verarbeiteten Daten des Bestimmungsziels ein Rauschen enthalten oder nicht, basierend auf der Ähnlichkeit,
wobei, hinsichtlich der Einheitswellenformdaten, die Datenverarbeitungseinheit konfiguriert ist zum:
Bestimmen, ob ein Potential eines Wendepunkts unmittelbar vor oder unmittelbar nach der Spitze der R-Welle eine Referenz überschreitet oder nicht oder ob ein Potential eines Wendepunkts unmittelbar vor oder unmittelbar nach der Spitze der S-Welle unterhalb einer Referenz liegt oder nicht, für das Erfassen eines Spitzenverlusts,
Generieren von Interpolationsdaten, in denen der Spitzenverlustteil interpoliert ist, und
Einstellen einer Wellenhöhe der Interpolationsdaten.

4. Elektrokardiogrammsignal-Analysesystem (3), das elektrokardiografische Informationen erhält und eine Verarbeitung durchführt, wobei das Elektrokardiogrammsignal-Analysesystem umfasst:
eine Eingabeeinheit (31), die konfiguriert ist zum Empfangen einer Eingabe der elektrokardiografischen Informationen,
eine Filterverarbeitungseinheit (32), die konfiguriert ist zum Anwenden eines Filters auf die elektrokardiografischen Informationen und zum Ausgeben von gefilterten elektrokardiografischen Informationen, wobei das Filter ein Hindurchgehen einer charakteristischen Frequenz erlaubt,
eine Einheitswellenformdaten-Extraktionseinheit (33), die konfiguriert ist zum Durchführen einer Segmentierung und zum Extrahieren von Einheitswellenformdaten basierend auf einer Spitze einer R-Welle oder einer S-Welle aus in den gefilterten elektrokardiografischen Informationen enthaltenen Wellenformdaten,
eine Datenverarbeitungseinheit (34), die konfiguriert ist zum Generieren von verarbeiteten Daten, in denen eine Wellenhöhe der Einheitswellenformdaten eingestellt ist,
eine Referenzdaten-Auswahleinheit (35), die konfiguriert ist zum Auswählen von Referenzdaten als einer Referenz eines Messzustands aus den verarbeiteten Daten,
eine Ähnlichkeitsberechnungseinheit (36), die konfiguriert ist zum Berechnen einer Ähnlichkeit zwischen den Referenzdaten und den verarbeiteten Daten eines Bestimmungsziels, und
eine Bestimmungseinheit (37), die konfiguriert ist zum Bestimmen, ob die elektrokardiografischen Informationen in Entsprechung zu den verarbeiteten Daten des Bestimmungsziels ein Rauschen enthalten oder nicht, basierend auf der Ähnlichkeit,
wobei die Datenverarbeitungseinheit (34) konfiguriert ist zum:
Invertieren einer Wellenhöhe der Einheitswellenformdaten oder der verarbeiteten Daten, und
Einstellen einer Wellenhöhe der invertierten Daten.

5. Elektrokardiogrammsignal-Analysesystem (3), das elektrokardiografische Informationen erhält und eine Verarbeitung durchführt, wobei das Elektrokardiogrammsignal-Analysesystem umfasst:
eine Eingabeeinheit (31), die konfiguriert ist zum Empfangen einer Eingabe der elektrokardiografischen Informationen,
eine Filterverarbeitungseinheit (32), die konfiguriert ist zum Anwenden eines Filters auf die elektrokardiografischen Informationen und zum Ausgeben von gefilterten elektrokardiografischen Informationen, wobei das Filter ein Hindurchgehen einer charakteristischen Frequenz erlaubt,
eine Einheitswellenformdaten-Extraktionseinheit (33), die konfiguriert ist zum Durchführen einer Segmentierung und zum Extrahieren von Einheitswellenformdaten basierend auf einer Spitze einer R-Welle oder einer S-Welle aus in den gefilterten elektrokardiografischen Informationen enthaltenen Wellenformdaten,
eine Datenverarbeitungseinheit (34), die konfiguriert ist zum Generieren von verarbeiteten Daten, in denen eine Wellenhöhe der Einheitswellenformdaten eingestellt ist,
eine Referenzdaten-Auswahleinheit (35), die konfiguriert ist zum Auswählen von Referenzdaten als einer Referenz eines Messzustands aus den verarbeiteten Daten,
eine Ähnlichkeitsberechnungseinheit (36), die konfiguriert ist zum Berechnen einer Ähnlichkeit zwischen den Referenzdaten und den verarbeiteten Daten eines Bestimmungsziels, und
eine Bestimmungseinheit (37), die konfiguriert ist zum Bestimmen, ob die elektrokardiografischen Informationen in Entsprechung zu den verarbeiteten Daten des Bestimmungsziels ein Rauschen enthalten oder nicht, basierend auf der Ähnlichkeit,
wobei die Datenverarbeitungseinheit (34) konfiguriert ist zum:
Invertieren der Einheitswellenformdaten oder der verarbeiteten Daten, und
Einstellen der Wellenhöhe der invertierten Daten.

6. Elektrokardiogrammsignal-Analysesystem nach einem der Ansprüche 1 bis 5, wobei:
die Ähnlichkeitsberechnungseinheit konfiguriert ist zum:
Berechnen der Ähnlichkeit zwischen den dynamisch zeitverzerrten Referenzdaten und den verarbeiteten Daten des Bestimmungsziels.

7. Elektrokardiogrammsignal-Analysesystem nach einem der Ansprüche 1 bis 6, wobei:
die Referenzdaten-Auswahleinheit konfiguriert ist zum Auswählen der Referenzdaten aus Bestimmungsdaten, die basierend auf elektrokardiografischen Informationen generiert wurden, die von einem Träger, der ein die elektrokardiografischen Informationen erhaltendes Messinstrument trägt, erhalten werden.

8. Elektrokardiogrammsignal-Analysesystem nach einem der Ansprüche 1 bis 6, wobei:
die Referenzdaten-Auswahleinheit konfiguriert ist zum Auswählen der Referenzdaten aus Bestimmungsdaten, die basierend auf elektrokardiografischen Informationen generiert werden, die von einer anderen Person als einem Träger, der ein die elektrokardiografischen Informationen erhaltendes Messinstrument trägt, erhalten werden.

## Revendications

1. Système (3) d'analyse de signal électrocardiographique qui acquiert des informations électrocardiographiques et effectue un traitement, le système d'analyse de signal électrocardiographique comprenant :
une unité d'entrée (31) configurée pour recevoir l'entrée des informations électrocardiographiques ;
une unité (32) de traitement par filtrage configurée pour appliquer un filtre aux informations électrocardiographiques et délivrer des informations électrocardiographiques filtrées, le filtre laissant passer une fréquence caractéristique ;
une unité (33) d'extraction de données de forme d'onde unitaire configurée pour effectuer une segmentation et extraire des données de forme d'onde unitaire sur la base d'un pic d'une onde R ou d'une onde S à partir de données de forme d'onde comprises dans les informations électrocardiographiques filtrées ;
une unité (34) de traitement de données configurée pour générer des données traitées dans lesquelles une hauteur d'onde des données de forme d'onde unitaire est ajustée ;
une unité (35) de sélection de données de référence configurée pour sélectionner, parmi les données traitées, des données de référence destinées à servir de référence d'un état de mesure ;
une unité (36) de calcul de similarité configurée pour calculer une similarité entre les données de référence et les données traitées d'une cible de détermination ; et
une unité (37) de détermination configurée pour déterminer si les informations électrocardiographiques correspondant aux données traitées de la cible de détermination comprennent ou non du bruit sur la base de la similarité,
dans lequel
concernant les données de forme d'onde unitaire, l'unité de traitement de données est configurée pour déterminer si un potentiel d'un point minimum situé immédiatement avant ou immédiatement après le pic de l'onde R dépasse une référence, ou si un potentiel d'un point maximum situé immédiatement avant ou immédiatement après le pic de l'onde S est inférieur à une référence, afin de détecter une perte de pic,
générer des données d'interpolation dans lesquelles la partie de perte de pic est interpolée, et
ajuster une hauteur d'onde des données d'interpolation.

2. Système (3) d'analyse de signal électrocardiographique qui acquiert des informations électrocardiographiques et effectue un traitement, le système d'analyse de signal électrocardiographique comprenant :
une unité d'entrée (31) configurée pour recevoir l'entrée des informations électrocardiographiques ;
une unité (32) de traitement par filtrage configurée pour appliquer un filtre aux informations électrocardiographiques et délivrer des informations électrocardiographiques filtrées, le filtre laissant passer une fréquence caractéristique ;
une unité (33) d'extraction de données de forme d'onde unitaire configurée pour effectuer une segmentation et extraire des données de forme d'onde unitaire sur la base d'un pic d'une onde R ou d'une onde S à partir de données de forme d'onde comprises dans les informations électrocardiographiques filtrées ;
une unité (34) de traitement de données configurée pour générer des données traitées dans lesquelles une hauteur d'onde des données de forme d'onde unitaire est ajustée ;
une unité (35) de sélection de données de référence configurée pour sélectionner, parmi les données traitées, des données de référence destinées à servir de référence d'un état de mesure ;
une unité (36) de calcul de similarité configurée pour calculer une similarité entre les données de référence et les données traitées d'une cible de détermination ; et une unité (37) de détermination configurée pour déterminer si les informations électrocardiographiques correspondant aux données traitées de la cible de détermination comprennent ou non du bruit sur la base de la similarité, dans lequel
concernant les données de forme d'onde unitaire, l'unité de traitement de données est configurée pour
déterminer si un potentiel d'un point maximum situé immédiatement avant ou immédiatement après le pic de l'onde R dépasse une référence, ou si un potentiel d'un point minimum situé immédiatement avant ou immédiatement après le pic de l'onde S est inférieur à une référence, afin de détecter une perte de pic,
générer des données d'interpolation dans lesquelles la partie de perte de pic est interpolée, et
ajuster une hauteur d'onde des données d'interpolation.

3. Système (3) d'analyse de signal électrocardiographique qui acquiert des informations électrocardiographiques et effectue un traitement, le système d'analyse de signal électrocardiographique comprenant :
une unité d'entrée (31) configurée pour recevoir l'entrée des informations électrocardiographiques ;
une unité (32) de traitement par filtrage configurée pour appliquer un filtre aux informations électrocardiographiques et délivrer des informations électrocardiographiques filtrées, le filtre laissant passer une fréquence caractéristique ;
une unité (33) d'extraction de données de forme d'onde unitaire configurée pour
effectuer une segmentation et extraire des données de forme d'onde unitaire sur la base d'un pic d'une onde R ou d'une onde S à partir de données de forme d'onde comprises dans les informations électrocardiographiques filtrées ;
une unité (34) de traitement de données configurée pour générer des données traitées dans lesquelles une hauteur d'onde des données de forme d'onde unitaire est ajustée ;
une unité (35) de sélection de données de référence configurée pour sélectionner, parmi les données traitées, des données de référence destinées à servir de référence d'un état de mesure ;
une unité (36) de calcul de similarité configurée pour calculer une similarité entre les données de référence et les données traitées d'une cible de détermination ; et
une unité (37) de détermination configurée pour déterminer si les informations électrocardiographiques correspondant aux données traitées de la cible de détermination comprennent ou non du bruit sur la base de la similarité, dans lequel
concernant les données de forme d'onde unitaire, l'unité de traitement de données est configurée pour déterminer si un potentiel d'un point d'inflexion situé immédiatement avant ou immédiatement après le pic de l'onde R dépasse une référence, ou si un potentiel d'un point d'inflexion situé immédiatement avant ou immédiatement après le pic de l'onde S est inférieur à une référence, afin de détecter une perte de pic,
générer des données d'interpolation dans lesquelles la partie de perte de pic est interpolée, et
ajuster une hauteur d'onde des données d'interpolation.

4. Système (3) d'analyse de signal électrocardiographique qui acquiert des informations électrocardiographiques et effectue un traitement, le système d'analyse de signal électrocardiographique comprenant :
une unité d'entrée (31) configurée pour recevoir l'entrée des informations électrocardiographiques ;
une unité (32) de traitement par filtrage configurée pour appliquer un filtre aux informations électrocardiographiques et délivrer des informations électrocardiographiques filtrées, le filtre laissant passer une fréquence caractéristique ;
une unité (33) d'extraction de données de forme d'onde unitaire configurée pour effectuer une segmentation et extraire des données de forme d'onde unitaire sur la base d'un pic d'une onde R ou d'une onde S à partir de données de forme d'onde comprises dans les informations électrocardiographiques filtrées ;
une unité (34) de traitement de données configurée pour générer des données traitées dans lesquelles une hauteur d'onde des données de forme d'onde unitaire est ajustée ;
une unité (35) de sélection de données de référence configurée pour sélectionner, parmi les données traitées, des données de référence destinées à servir de référence d'un état de mesure ;
une unité (36) de calcul de similarité configurée pour calculer une similarité entre les données de référence et les données traitées d'une cible de détermination ; et
une unité (37) de détermination configurée pour déterminer si les informations électrocardiographiques correspondant aux données traitées de la cible de détermination comprennent ou non du bruit sur la base de la similarité, dans lequel
l'unité (34) de traitement de données est configurée pour inverser une hauteur d'onde des données de forme d'onde unitaire ou des données traitées, et
ajuster une hauteur d'onde des données inversées.

5. Système (3) d'analyse de signal électrocardiographique qui acquiert des informations électrocardiographiques et effectue un traitement, le système d'analyse de signal électrocardiographique comprenant :
une unité d'entrée (31) configurée pour recevoir l'entrée des informations électrocardiographiques ;
une unité (32) de traitement par filtrage configurée pour appliquer un filtre aux informations électrocardiographiques et délivrer des informations électrocardiographiques filtrées, le filtre laissant passer une fréquence caractéristique ;
une unité (33) d'extraction de données de forme d'onde unitaire configurée pour effectuer une segmentation et extraire des données de forme d'onde unitaire sur la base d'un pic d'une onde R ou d'une onde S à partir de données de forme d'onde comprises dans les informations électrocardiographiques filtrées ;
une unité (34) de traitement de données configurée pour générer des données traitées dans lesquelles une hauteur d'onde des données de forme d'onde unitaire est ajustée ;
une unité (35) de sélection de données de référence configurée pour sélectionner, parmi les données traitées, des données de référence destinées à servir de référence d'un état de mesure ;
une unité (36) de calcul de similarité configurée pour calculer une similarité entre les données de référence et les données traitées d'une cible de détermination ; et
une unité (37) de détermination configurée pour déterminer si les informations électrocardiographiques correspondant aux données traitées de la cible de détermination comprennent ou non du bruit sur la base de la similarité, dans lequel
l'unité (34) de traitement de données est configurée pour inverser le temps des données de forme d'onde unitaire ou des données traitées, et
ajuster une hauteur d'onde des données inversées.

6. Système d'analyse de signal électrocardiographique selon l'une quelconque des revendications 1 à 5, dans lequel
l'unité de calcul de similarité est configurée pour calculer une similarité entre les données de référence ayant fait l'objet d'un alignement temporel dynamique et les données traitées de la cible de détermination.

7. Système d'analyse de signal électrocardiographique selon l'une quelconque des revendications 1 à 6, dans lequel
l'unité de sélection de données de référence est configurée pour sélectionner les données de référence parmi des données de détermination générées sur la base d'informations électrocardiographiques acquises auprès d'un porteur portant un instrument de mesure qui acquiert les informations électrocardiographiques.

8. Système d'analyse de signal électrocardiographique selon l'une quelconque des revendications 1 à 6, dans lequel
l'unité de sélection de données de référence est configurée pour sélectionner les données de référence parmi des données de détermination générées sur la base d'informations électrocardiographiques acquises auprès d'un sujet autre qu'un porteur portant un instrument de mesure qui acquiert les informations électrocardiographiques.
